(19) Europäisches Patentamt
European Patent Office
Office européen des brevets

(11) EP 0 485 748 B1

(12) **EUROPEAN PATENT SPECIFICATION**

(45) Date of publication and mention of the grant of the patent:
**24.07.1996 Bulletin 1996/30**

(51) Int. Cl.$^6$: **C08B 37/10**, C08B 37/08, A61K 31/725

(21) Application number: **91117517.2**

(22) Date of filing: **14.10.1991**

(54) **Salts of glycosaminoglycans with esters of aminoacids, preparation thereof and pharmaceutical formulations containing them**

Salze von Glykosaminoglykanen mit Aminosäureester, ihre Herstellung sowie sie enthaltende pharmazeutische Zusammensetzungen

Sels de glycosaminoglycanes avec des esters d'acides aminés, leur préparation et compositions pharmaceutiques les contenant

(84) Designated Contracting States:
**BE DE ES FR GB NL**

(30) Priority: **13.11.1990 IT 372890**

(43) Date of publication of application:
**20.05.1992 Bulletin 1992/21**

(73) Proprietor: **ALFA WASSERMANN S.p.A.**
**I-65020 Alanno Scalo (Pescara) (IT)**

(72) Inventors:
- **Cristofori, Manlio**
  **I-40127 Bologna (IT)**
- **Marchi, Egidio**
  **I-40033 Casalecchio di Reno (Bologna) (IT)**
- **Rotini, Leone Gabriele**
  **I-40133 Bologna (IT)**

(74) Representative: **Kraus, Walter, Dr. et al**
**Patentanwälte Kraus, Weisert & Partner**
**Thomas-Wimmer-Ring 15**
**D-80539 München (DE)**

(56) References cited:
**WO-A-89/12070**
**US-A- 3 088 868**
**US-A- 3 506 642**
**US-A- 4 105 760**

- **THROMBOSIS RESEARCH vol. 1, no. 2, 30 April 1972, NEW YORK pages 173 - 181 I. DANISHEFSKY ET AL. 'HEPARIN DERIVATIVES PREPARED BY MODIFICATION OF THE URONIC ACID CARBOXYL GROUPS.'**

Remarks:
The file contains technical information submitted after the application was filed and not included in this specification

EP 0 485 748 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.13.0/3.4

## Description

### BACKGROUND OF THE INVENTION

The glycosaminoglycans are products of natural origin, obtained from tissues of animal origin, made by heterogeneous mixtures of chains of polysaccharides sulfated in different ways having a very wide range of molecular weights, varying from few thousands to some tens of thousands of Daltons.

Heparin, mainly made by units containing D-glucosamine and L-iduronic or D-glucuronic acid sulfated in different ways, having a molecular weight ranging from 6000 to 30000 Daltons, generally used as anticoagulant and antithrombotic drug as sodium, potassium, calcium or magnesium salt, is the most known among them.

Low molecular weight heparins, derivatives having a smaller degree of polymerization, with molecular weights ranging from 1500 to 8000 Daltons, having therapeutical activities similar to those of the heparin, are obtained from heparin by chemical or enzymatic treatments.

The chondroitins are other glycosaminoglycans extracted from tissues of animal origin, one of which, previoulsy known as chondroitin sulfate B, is the dermatan sulfate having antithrombotic and antihyperlipoproteinemic activity, from which, like in the case of heparin, it is possible to obtain low molecular weight fractions ranging from 2000 to 8000 Daltons.

Glucuronylglycosaminoglycan sulfate, known under the name sulodexide (International Nonproprietary Name), having antithrombotic and antiatherosclerotic activity, is another substance pertaining to this class of drugs.

All these polysaccharides, as salts of alkali or alkali-earth metals (sodium, potassium, calcium and magnesium) were extensively studied in the prevention and therapy of many pathologies of thrombotic or atherosclerotic origin.

Nevertheless, their therapeutic use is restricted by the fact that these salts have to be parenterally administered owing to their scarce absorption by oral administration.

For quite a time remarkable efforts are carried out to find adjuvant substances or derivatives or pharmaceutical formulations which can be able to make the glycosaminoglycans orally absorbable, in view of their great therapeutical interest in the prevention and therapy of atherosclerotic and thrombotic pathologies.

At the beginning they tried to solve the problem by using adjuvant substances like EDTA [(Tidball et al., Proc. Soc. Exp. Biol. Med. 111, 713-5, (1962)], dimethylsulfoxide and diethylsulfone [Koh T.Y., Can. J. Biochem., 47, 951-4 (1969)], nitrilotriacetic acid [Jarret et al., Thromb. Diath. Haemorrh., 25, 187-200, (1971)] or citric acid [Sue T.K. et al., Can. J. Physiol. Pharmacol., 54, (4), 613-7, (1976)].

Engel R.H. and Riggi S.J. contemporaneously tried to help the oral absorption of the glycosaminoglycans by adding some surfactants, mainly sulfated or sulfonated surfactants, which cause the formation of emulsions that aid the absorption in the experimental animals [J. Pharm. Sci.,58, 706-10 and 1372-5, (1969)].

Another way was that of preparing salts and complexes with weakly basic organic substances, like amines, or amphoteric substances, like amides or aminoacids, as reported in US patents 3506642 and 3577534.

More recently they tried to aid the absorption of the glycosaminoglycans by using as vehicle appropriate pharmaceutical formulations containing liposomes (Masaharu Ueno et al., Chem. Pharm. Bull., 30, (6), 2245-7, (1982), belgian patent BE 860011, french patent FR 2492259), or by making complexes with ammonium quarternary bases (international publications PCT WO 85/05362, US patents 4510135 and 4654327).

In spite of all these attempts, oral formulations of glycosaminoglycans for the therapy of the thrombotic and atherosclerotic pathologies are not commercialized yet.

This is probably due to the fact that the till now described formulations contain substances which are not tolerated by the human body, mainly in the long run, and/or these formulations do not possess the necessary stability.

Therefore it is still necessary to find pharmaceutical formulations containing glycosaminoglycans, suitable for the oral absorption.

### SUMMARY OF THE INVENTION

New salts of glycosaminoglycans, orally absorbable prepared by salification of glycosaminoglycans, under acidic form after treatment with ionic exchange resins, with esters of aminoacids of formula

$$R_1 - \underset{\displaystyle NH_2}{\underset{|}{CH}} - COOR_2 \qquad I$$

wherein $R_1$ represents hydrogen, straight or branched $C_1$-$C_6$-alkyl, aryl, substituted or unsubstituted arylalkyl and $R_2$ represents straight or branched $C_1$-$C_{12}$-alkyl, cycloalkyl, aryl or arylalkyl, and isolated by freeze-drying, are the object of the present invention.

Pharmaceutical formulations containing said salts are a further object of the present invention.

Preferred pharmaceutical formulations are those orally administrable, mainly gastrorestistant capsules and tablets, suitable for releasing the active principle in the more proper place for a better absorption, i.e. in the duodenal and intestinal zones, so avoiding the degradation caused by the gastric juices.

The use, as glycosaminoglycans, of the heparin and of its low molecular weight fractions, obtained according to well known methods of chemical or enzymatic depolymerization, of the glucuronylglycosaminoglycan sulfate known as sulodexide, of the dermatan sulfate and of its low molecular weight fractions, constitutes a preferred aspect of the present invention.

Another preferred aspect of the present invention is that in which the esters of aminoacids of formula

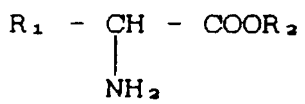

I

used for the salification of the glycosaminoglycans are those wherein $R_1$ represents hydrogen or substituted or unsubstituted arylalkyl and $R_2$ represents straight or branched $C_1$-$C_6$-alkyl, or arylalkyl.

Methyl, ethyl, isopropyl, tert-butyl, isoamyl and benzyl esters of glycine, phenylalanine and tyrosine are preferred among these compounds of formula I.

The process of preparation of the salts of the glycosaminoglycans with the esters of aminoacids of formula I initially foresees the removal of the alkaline or alkaline-earth cation, generally sodium, of the glycosaminoglycan and its substitution by a $H^+$ ion to obtain the acidic, not salified, form of the glycosaminoglycan. This removal is obtained by percolating an aqueous solution of an alkaline or alkaline-earth salt of glycosaminoglycan through a chromatographic column containing a cationic resin activated in $H^+$ form, preferably an AG 50W-X8 resin in $H^+$ form, and by eluting with distilled water until the eluate becomes neutral. The eluate containing the glycosaminoglycan in acidic form is directly poured into an alcoholic solution of an ester of aminoacid of formula I and the resulting solution is partially evaporated under vacuum until complete elimination of the alcohol used to dissolve the ester of the aminoacid. The alcohol used is an alcohol containing from 1 to 3 carbon atoms, preferably methanol which has the advantage of having the lowest boiling point.

The resulting aqueous solution is freeze-dried obtaining the desired salt with quantitative yield.

The amounts of esters of aminoacids used to salify the glycosaminoglycans are equimolecular in respect to the milliequivalents of acidic groups, sulfates and carboxyls, for each gram of glycosaminoglycan, determined by potentiometric analysis.

The so obtained salts were characterized according to some chemical-physical parameters, like the specific rotatory power and the infrared and ultraviolet spectra, and their chemical-physical characteristics are listed in the following table 1.

## TABLE 1

### Chemical-physical characteristics of salts of glycosaminoglycans with aminoesters

| Compound described in example | Rotatory Power 589 nm | Rotatory Power 546 nm | I.R. Spectrum ($cm^{-1}$) | U.V. Spectrum (nm) |
|---|---|---|---|---|
| 1 | +25.5 | +32.1 | 1230,1505,1610, 1730,3100-3400 | 223 274 |
| 2 | +26.8 | +32.8 | 1050,1150,1250, 1380,1450,1520, 1620,1740,3000, 3450 | 223 273 |
| 3 | +29.5 | +42.1 | 1220,1510,1610, 1730,3000-3500 | 224 274 |
| 4 | +27.4 | +36.5 | 1240,1510,1610, 1730,3000-3500 | 224 274 |
| 5 | +25.8 | +34.1 | 1220,1400,1490, 1620,1740,3000-3500 | 252 256 262 267 |
| 6 | +38.9 | +47.4 | 1260,1610,1740, 2900,3450 | 258 |
| 7 | +25.3 | +34.4 | 1240,1510,1610, 1740,3000-3400 | 224 274 |
| 8 | +24.6 | +30.2 | 1040,1100,1150, 1250,1380,1450, 1500,1620,1740 3100,3450 | 263 257 251 |

continues...

| Compound described in example | Rotatory Power | | I.R. Spectrum | U.V. Spectrum |
|---|---|---|---|---|
| | 589 nm | 546 nm | ($cm^{-1}$) | (nm) |
| 9 | +27.8 | +35.0 | 1260,1620, 1740,3000-3500 | 223 272 |
| 10 | +22.6 | +25.6 | 1250,1610,1740 3000-3500 | 223 273 |
| 11 | +40.7 | +48.6 | 1260,1620,1740, 3000-3500 | 224 272 |

The rotatory powers were determined in 20% (v/v) aqueous methanol at 1% concentration and at 20°C.

The I.R. spectra were carried out in KBr with a Perkin-Elmer 281B spectrophotometer.

The U.V. spectra were carried out in 20% (v/v) aqueous methanol with a double beam Perkin-Elmer 552 spectrophotometer.

The salts of the glycosaminoglycans object of the present invention show the same pharmacological characteristics as the corresponding alkaline and alkaline-earth salts and therefore the use of these salts and of the pharmaceutical formulations containing them in the prevention and treatment of the thrombotic and atherosclerotic pathologies constitutes another aspect of the present invention.

The so obtained and characterized salts of the glycosaminoglycans with the esters of the aminoacids of formula I were submitted to some pharmacological tests on animals to verify their true absorption in respect of the non absorption of the sodium salts of the same glycosaminoglycans.

In particular, the antithrombotic activity and the variations of three parameters generally used to define the activity of the glycosaminoglycans were tested and therefore the following tests were carried out:

1. Dosage of the clearing-factor with Ediol test as described from Bianchini et al., Biochem. Exp. Biol., 10, 243-7 (1972).
2. Dosage of the activated partial thromboplastin time (APTT) according to the coagulometric method of Larrieu M.T. et al., Rev. Hemat., 12, 199, (1957), by using the Boehringer's analytic kit.
3. Dosage of the inhibition of the activated tenth factor (AXa) according to the coagulometric method of Yin E.T., et al., Lab. Clin. Med., 81, 298-310 (1973), by using the Sigma's analytic kit.

The antithrombotic activity was evaluated according to the method of Reyers S., et al., Thromb. Res., 18, 669-674 (1980), which consists of the ligature of the inferior vena cava, subsequent stasis and formation of the thrombus.

The bioavailability biological tests were carried out on Sprague-Dawley male rats having a body weight between 280 g and 300 g, shared in groups of six animals, fasted 16 hours before the start of the test.

The salts of the glycosaminoglycans were directly administered in the duodenum, according to the method described by Engel R.H. et al., Proc. Soc. Exptl. Biol. Med., 129, (5), 772-7, (1968) at the dose of 50 mg of active principle for each Kg of body weight of the experimental animals, dissolved in a volume of distilled water or suspended in a volume of vegetable oil equal to 0.1 ml for each 100 g of body weight. The same volume of the liquid used for the administration of the active principle was given to the control group.

The animals were sacrificed under general anaesthesia from ethyl ether and the blood was directly taken from the heart at the times of 15, 30 and 60 minutes for the treated groups, while only a taking after 30 minutes was carried out for the control group and the time was indicated equal to zero in table 2 because the vehicle, distilled water or oil, does not exert any influence on the biological parameters.

The evaluations of the previoulsy described biological parameters were carried out on the plasma obtained by addition of sodium citrate. The values relating to the variation of the parameters of the clearing-factor, of the activated thromboplastin time (APTT) and of the inhibition of the activated tenth factor (AXa) produced by the salts object of the present invention in comparison with the sodium salts of the glycosaminoglycans are reported in the following table 2.

The numerical values clearly show the effect obtained with the administration of the salts object of the present invention in comparison with no effect obtained with the administration of the corresponding sodium salts.

## TABLE 2

Effect on clearing-factor, APTT and AXa of the salts of the glycosaminoglycans according to the present invention in comparison with the corresponding sodium salts..

| Active Principle | Time (min) | Clearing-factor O.D. % after 30 minutes of incubation $\bar{x}$ ±s.e. | APTT (sec) $\bar{x}$ ±s.e. | AXa (sec) $\bar{x}$ ±s.e. |
|---|---|---|---|---|
| Low molecular weight heparin (LMWH) R 82812 | 0 | 100.9 ±1.6 | 19.5 ±0.2 | 68.0 ±1.2 |
| | 15 | 99.6 ±2.2 | 20.3 ±0.3 | 70.1 ±0.8 |
| | 30 | 97.9 ±1.5 | 20.6 ±1.1 | 69.3 ±1.2 |
| | 60 | 98.8 ±1.5 | 19.7 ±0.6 | 68.6 ±1.3 |

continues...

| Active Principle | Time (min) | Clearing-factor O.D. % after 30 minutes of incubation $\bar{x}$ ±s.e. | APTT (sec) $\bar{x}$ ±s.e. | AXa (sec) $\bar{x}$ ±s.e. |
|---|---|---|---|---|
| Example 1 | 0 | 97.7 ±0.7 | 23.2 ±0.7 | 56.1 ±0.5 |
| | 15 | 58.2 ±3.3 | 37.9 ±3.5 | 72.1 ±5.2 |
| | 30 | 71.5 ±4.4 | 27.5 ±1.1 | 63.4 ±1.2 |
| | 60 | 85.7 ±1.7 | 33.0 ±2.0 | 66.4 ±1.7 |
| Example 2 | 0 | 101.1 ±0.5 | 17.9 ±0.9 | 56.8 ±1.8 |
| | 15 | 56.4 ±1.9 | 59.7 ±8.2 | 77.5 ±2.8 |
| | 30 | 59.8 ±4.1 | 141.3 ±3.8 | 71.7 ±2.2 |
| | 60 | 68.1 ±3.5 | 41.3 ±2.8 | 70.5 ±1.2 |
| Example 3 | 0 | 100.4 ±0.2 | 22.2 ±1.4 | 75.4 ±2.6 |
| | 15 | 68.6 ±5.1 | 30.2 ±1.9 | 83.0 ±1.9 |
| | 30 | 74.4 ±2.4 | 31.4 ±2.4 | 85.3 ±1.6 |
| | 60 | 74.4 ±3.8 | 30.2 ±1.9 | 78.9 ±2.8 |
| Example 4 | 0 | 107.8 ±7.3 | 22.8 ±1.7 | 70.0 ±1.2 |
| | 15 | 69.4 ±3.7 | 23.8 ±1.7 | 73.1 ±2.6 |
| | 30 | 75.4 ±6.0 | 23.8 ±1.7 | 71.2 ±0.8 |
| | 60 | 93.9 ±2.7 | 20.8 ±0.8 | 71.0 ±1.3 |

continues...

| Active Principle | Time (min) | Clearing-factor O.D. % after 30 minutes of incubation $\bar{x}$ ±s.e. | APTT (sec) $\bar{x}$ ±s.e. | AXa (sec) $\bar{x}$ ±s.e. |
|---|---|---|---|---|
| Example 5 | 0 | 97.8 ±1.4 | 19.9 ±0.7 | 78.8 ±1.7 |
| | 15 | 41.4 ±4.9 | 81.8 ±7.2 | 96.1 ±2.9 |
| | 30 | 52.8 ±2.5 | 56.3 ±3.7 | 91.2 ±2.5 |
| | 60 | 81.4 ±4.3 | 49.4 ±3.9 | 88.0 ±1.9 |
| Example 6 | 0 | 99.6 ±0.5 | 23.7 ±2.0 | 80.5 ±2.6 |
| | 15 | 62.5 ±4.1 | 44.4 ±2.3 | 106.9 ±2.1 |
| | 30 | 53.5 ±1.7 | 80.0 ±3.9 | 104.0 ±2.6 |
| | 60 | 76.8 ±2.7 | 46.1 ±4.6 | 101.0 ±2.6 |
| Example 7 | 0 | 99.6 ±0.5 | 23.7 ±2.0 | 80.5 ±2.6 |
| | 15 | 62.5 ±4.1 | 44.4 ±2.3 | 106.9 ±2.1 |
| | 30 | 53.5 ±1.7 | 80.0 ±3.9 | 104.0 ±2.6 |
| | 60 | 76.8 ±2.7 | 46.1 ±4.6 | 101.0 ±2.6 |
| Example 8 | 0 | 101.5 ±1.1 | 24.6 ±2.1 | 72.6 ±2.5 |
| | 15 | 56.9 ±2.8 | 48.4 ±2.7 | 82.0 ±2.8 |
| | 30 | 57.5 ±4.6 | 51.8 ±3.3 | 93.1 ±2.9 |
| | 60 | 76.2 ±3.4 | 51.1 ±3.6 | 86.8 ±2.0 |

continues...

| Active Principle | Time (min) | Clearing-factor O.D. % after 30 minutes of incubation x̄ ±s.e. | APTT (sec) x̄ ±s.e. | AXa (sec) x̄ ±s.e. |
|---|---|---|---|---|
| glucuronyl-glycosamino glycan sulfate SULODEXIDE | 0 | 94.7 ±0.8 | 23.3 ±0.8 | 69.9 ±1.2 |
| | 15 | 87.6 ±4.5 | 22.9 ±1.5 | 71.2 ±1.2 |
| | 30 | 93.5 ±2.3 | 22.0 ±2.3 | 68.1 ±1.5 |
| | 60 | 93.4 ±2.2 | 22.6 ±1.5 | 67.2 ±0.8 |
| Example 9 | 0 | 104.8 ±0.3 | 19.6 ±0.6 | 78.6 ±6.4 |
| | 15 | 90.1 ±3.9 | 30.6 ±2.6 | 91.0 ±7.6 |
| | 30 | 93.4 ±1.3 | 31.1 ±3.1 | 89.7 ±3.5 |
| | 60 | 92.8 ±3.6 | 36.1 ±7.2 | 83.3 ±3.8 |
| Injectable sodium heparin 158 N26 | 0 | 100.7 ±0.4 | 23.1 ±0.6 | 70.1 ±1.3 |
| | 15 | 99.4 ±2.5 | 22.4 ±0.3 | 68.2 ±2.4 |
| | 30 | 99.1 ±1.1 | 23.6 ±0.1 | 70.3 ±1.6 |
| | 60 | 98.5 ±2.3 | 24.1 ±0.4 | 71.1 ±1.4 |
| Example 11 | 0 | 103.4 ±0.6 | 28.4 ±1.8 | 78.6 ±2.3 |
| | 15 | 85.3 ±4.2 | 111.4 ±14.4 | 90.1 ±4.6 |
| | 30 | 99.4 ±2.5 | 66.3 ±12.8 | 84.4 ±1.0 |
| | 60 | 105.5 ±0.2 | 25.4 ±2.1 | 78.2 ±2.4 |

The antithrombotic activity was evaluated, as reported in the following table 3, for the salt of the low molecular weight dermatan sulfate with the tert-butyl ester of the tyrosine described in example 10, administering by intraduodenal route an oily suspension containing a dose of active principle equal to 200 mg for each Kg of body weight of the experimental animals. The data reported in the table, in comparison with the untreated control animals, unambiguously show the high degree of antithrombotic protection offered by one of the salts of the present invention and therefore its bioavailability.

TABLE 3

| Antithrombotic activity in the rat | | | | | |
|---|---|---|---|---|---|
| Active Principle | Time (min) | Number of animals | Number of animals with thrombi | Protection % | Thrombi weight ($\bar{x}$ ±s.e.) |
| No active principle | 15 | 8 | 8 | 0 | 4.34 ±0.6 |
| | 30 | 8 | 8 | 0 | 3.25 ±0.08 |
| Example 10 | 15 | 8 | 2 | 75 | 0.38 ±0.47 |
| | 30 | 8 | 0 | 100 | 0 |

A further object of the present invention is constituted by pharmaceutical formulations containing the salts of the glycosaminoglycans with the esters of the aminoacids of formula

$$R_1 - \underset{\displaystyle NH_2}{\underset{|}{CH}} - COOR_2 \qquad\qquad I$$

wherein $R_1$ and $R_2$ have the before mentioned meanings.

The pharmaceutical formulations orally administrable are those preferred according to the present invention. Said pharmaceutical formulations were expressly studied to permit to the active principle to unaltered cross the gastric juices and to be released in the duodenal and intestinal zones, so assuring an optimal absorption of the salts object of the present invention.

Capsules and tablets containing therapeutically effective amounts of salts according to the invention, preferably from 50 mg to 500 mg, suitably protected against the gastric juices of the stomach and apt to release the active principle in a pH range corresponding to that of the duodenal and intestinal zones of absorption, are preferred examples of said pharmaceutical formulations.

Many additives can be advantageously used for the gastroresistent enterosoluble coating of the pharmaceutical formulations preferred according to the present invention, tablets and capsules of soft or hard gelatine. The phthalates of polyvinyl and of cellulose, the copolymers and the esters of the metacrylic acid can be advantageously used. Within the scope of the present invention, the polyvinylacetophthalate is the preferred additive with the addition of plasticizers like the polyethylene glycole and the diethylphthalate necessary in order to improve the flexibility and the elasticity of the protective film.

The protection is effected by means of two consecutive coatings, the first coating is carried out in coating pan by using hydroxypropylmetylcellulose as main coating agent, together with polyethylene glycole, titanium dioxide and talc in a 22:1 mixture of 95% ethyl alcohol and water, while the second coating is carried out by sprying a solution made by polyvinylacetophthalate, diethylphthalate and stearic acid in 95% ethyl alcohol.

The salts object of the present invention can advantageously be administered also by means of the typical pharmaceutical formulations of the glycosaminoglycans, like for instance the parenteral formulations, as in the case of the corresponding alkaline and alkaline-earth salts.

The following glycosaminoglycans were used as starting compounds in the hereinafter examples:

a) Injectable sodium heparin, 158N26 batch, having an anticoagulant activity equal to 168 I.U./mg, supplied by the firm Opocrin (Italy).
b) Sodium low molecular weight heparin (LMWH), R82812 batch, obtained by depolymerization of sodium heparin by means of cupric acetate and hydrogen peroxide according to the method described in the european publication EP 0121067, having an average molecular weight equal to 4000 Daltons and an anticoagulant activity equal to 78 I.U./mg, supplied by the firm Opocrin (Italy).
c) Low molecular weight dermatan sulfate (DES-LMWH), OP 370L batch, having an average molecular weight equal to 5600 Daltons and a title equal to 1.4 APTT Units/mg and to 10 I.U. AXa/mg, supplied by the firm Opocrin (Italy).
d) Glucuronylglycosaminoglycan sulfate, known as sulodexide (INN), having a title equal to 3.7 APTT Units/mg and to 82 I.U. AXa/mg, supplied by the firm Alfa Wassermann (Italy).

The use of the above glycosaminoglycans only helps to illustrate the invention and has not to be considered as a limitation of the invention that can be effected with any other kind of glycosaminoglycan independently from its origin and from the greater or lesser degree of polymerization or sulfation.

**EXAMPLE 1**

Salt of low molecular weight heparin with tyrosine ethyl ester

A solution containing 2.00 g of low molecular weight heparin R82812 in 20 ml of distilled water is percolated through a chromatographic column containing 30 ml of cationic resin AG 50W-X8 under $H^+$ form, eluting with distilled water until neutral reaction of the eluate that is directly collected on a solution containing 2.05 g of tyrosine ethyl ester in 5 ml of methyl alcohol. The resulting hydroalcoholic solution is partly evaporated under vacuum until elimination of the methyl alcohol and the resulting aqueous solution is freeze-dried thus obtaining 3.76 g of salt.

**EXAMPLE 2**

Salt of low molecular weight heparin with tyrosine tert-butyl ester

By working in accordance with the same conditions set forth in example 1 and by using 2.33 g of tyrosine tert-butyl ester dissolved in 30 ml of methylalcohol, 4.04 g of salt are obtained.

**EXAMPLE 3**

Salt of low molecular weight heparin with tyrosine isoamyl ester

By working in accordance with the same conditions set forth in example 1 and by using 2.46 g of tyrosine isoamyl ester dissolved in 70 ml of methyl alcohol, 4.17 g of salt are obtained.

**EXAMPLE 4**

Salt of low molecular weight heparin with tyrosine isopropyl ester

By working in accordance with the same conditions set forth in example 1 and by using 2.19 g of tyrosine isopropyl ester dissolved in 80 ml of methyl alcohol, 3.92 g of salt are obtained.

**EXAMPLE 5**

Salt of low molecular weight heparin with glycine benzyl ester

By working in accordance with the same conditions set forth in example 1 and by using 1.62 g of glycine benzyl ester dissolved in 60 ml of methyl alcohol, 3.35 g of salt are obtained.

**EXAMPLE 6**

Salt of low molecular weight heparin with phenylalanine tert-butyl ester

By working in accordance with the same conditions set forth in example 1 and by using 2.53 g of phenylalanine tert-butyl ester dissolved in 70 ml of methyl alcohol, 4.35 g of salt are obtained.

**EXAMPLE 7**

Salt of low molecular weight heparin with tyrosine methyl ester

By working in accordance with the same conditions set forth in example 1 and by using 1.92 g of tyrosine methyl ester dissolved in 10 ml of methyl alcohol, 3.65 g of salt are obtained.

**EXAMPLE 8**

Salt of low molecular weight heparin with phenylalanine isopropyl ester

By working in accordance with the same conditions set forth in example 1 and by using 2.03 g of phenylalanine isopropyl ester dissolved in 70 ml of methyl alcohol, 3.80 g of salt are obtained.

**EXAMPLE 9**

Salt of glucuronylglycosaminoglycan sulfate with tyrosine tert-butyl ester

By working in accordance with the same conditions set forth in example 1 and by using 2.00 g of glucuronylglycosaminoglycan sulfate known as sulodexide (INN) and 2.20 g of tyrosine tert-butyl ester dissolved in 60 ml of methyl alcohol, 3.85 g of salt are obtained.

**EXAMPLE 10**

Salt of low molecular weight dermatan sulfate with tyrosine tert-butyl ester

By working in accordance with the same conditions set forth in example 1 and by using 3.00 g of low molecular weight dermatan sulfate OP 370L and 2.57 g of tyrosine tert-butyl ester dissolved in 80 ml of methyl alcohol, 5.20 g of salt are obtained.

**EXAMPLE 11**

Salt of heparin with tyrosine tert-butyl ester

By working in accordance with the same conditions set forth in example 1 and by using 2.00 g of heparin sodium salt and 2.18 g of tyrosine tert-butyl ester dissolved in 60 ml of methyl alcohol, 3.95 g of salt are obtained.

**EXAMPLE 12**

Gastroresistant hard gelatine capsules containing the salt of low molecular weight heparin with tyrosine tert-butyl ester

| Composition of each capsule: | |
|---|---|
| - Salt of low molecular weight heparin with tyrosine tert-butyl ester | 200 mg |

| Capsule coating: | |
|---|---|
| - Hydroxypropylmethylcellulose | 10.5 mg |
| - Polyethylene glycole 6000 | 0.6 mg |
| - Titanium dioxide | 2.4 mg |
| - Talc | 2.4 mg |
| - Polyvinylacetophthalate | 24.0 mg |
| - Diethylphthalate | 2.4 mg |
| - Stearic acid | 2.4 mg |

The salt of low molecular weight heparin with tyrosine tert-butyl ester is encapsuled in type 0 hard gelatine capsules that are first coated in coating pan with a mixture made by hydroxypropylmethylcellulose, polyethylene glycole 6000, titanium dioxide and talc in a mixture of 95% ethyl alcohol and water in 22:1 volumetric ratio. Subsequently a second gastroresistant coating is carried out by sprying a mixture of polyvinylacetophthalate, diethylphthalate and stearic acid dissolved in 95% ethyl alcohol.

The gastroresistant hard gelatine capsules containing the salts of the glycosaminoglycans with the esters of the aminoacids described in examples 1, 3, 4, 5, 6, 7, 8, 9, 10 and 11 are obtained in the same manner.

**EXAMPLE 13**

Gastroresistant soft gelatine capsules containing the salt of low molecular weight heparin with glycine benzyl ester

| Composition of each capsule: | |
|---|---|
| - Salt of low molecular weight heparin with glycine benzyl ester | 200 mg |
| - Caprilo-capric glycerides | 380 mg |

| Capsule coating: | |
|---|---|
| - Hydroxypropylmethylcellulose | 10.5 mg |
| - Polyethylene glycole 6000 | 0.6 mg |
| - Titanium dioxide | 2.4 mg |
| - Talc | 2.4 mg |
| - Polyvinylacetophthalate | 24.0 mg |
| - Diethylphthalate | 2.4 mg |
| - Stearic acid | 2.4 mg |

The salt of the low molecular weight heparin with the glycine benzyl ester is mixed with the caprilo-capric glycerides in a cylinder mill and the resulting mixture is shared in oval type 10 soft gelatine capsules that are coated in coating pan with a mixture made by hydroxypropylmethylcellulose, polyethylene glycole 6000, titanium dioxide and talc in 95% ethyl alcohol and water in 22:1 volumetric ratio. Subsequently a second gastroresistant coating is carried out by sprying a mixture of polyvinylacetophthalate, diethylphthalate and stearic acid dissolved in 95% ethyl alcohol.

The gastroresistant soft gelatine capsules containing the salts of the glycosaminoglycans with the esters of the aminoacids described in examples 1, 3, 4, 5, 6, 7, 8, 9, 10 and 11 are obtained in the same manner.

**EXAMPLE 14**

Gastroresistant tablets containing the salt of glucuronylglycosaminoglycan sulfate with tyrosine tert-butyl ester

| Composition of each tablet: | |
|---|---|
| - Salt of glucuronylglycosaminoglycan sulfate (sulodexide) with tyrosine tert-butyl ester | 200 mg |
| - Maize starch | 93.8 mg |
| - Lactose | 81.5 mg |
| - Microgranular cellulose | 300 mg |
| - Polyvinylpyrrolidone CL | 100 mg |
| - Magnesium stearate | 10 mg |

| Tablet coating: | |
|---|---|
| - Hydroxypropylmethylcellulose | 14 mg |
| - Polyethylene glycole 6000 | 0.4 mg |
| - Titanium dioxide | 3.2 mg |
| - Talc | 3.2 mg |
| - Polyvinylacetophthalate | 30 mg |
| - Diethylphthalate | 3 mg |
| - Stearic acid | 3 mg |

The salt of glucuronylglycosaminoglycan sulfate with tyrosine tert-butyl ester is mixed with maize starch and lactose and sifted on a sieve equipped with meshes having a side of 1 mm.

The so obtained granulate is mixed with microgranular cellulose, polyvinylpyrrolidone CL and magnesium stearate and is tabletted. The obtained tablets are coated in coating pan by means of a first film made by a mixture of hydroxypropylmethylcellulose, polyethylene glycole 6000, titanium dioxide and talc in 95% ethyl alcohol and water in 22:1 volumetric ratio. Subsequently a second gastroresistant coating is carried out by sprying a mixture of polyvinylacetophthalate, diethylphthalate and stearic acid dissolved in 95% ethyl alcohol.

The gastroresistant tablets containing the salts of the glycosaminoglycans with the esters of the aminoacids described in examples 1, 2, 3, 4, 5, 6, 7, 8, 10 and 11 are obtained in the same manner.

**EXAMPLE 15**

Gastroresistant tablets containing the salt of low molecular weight dermatan sulfate with tyrosine tert-butyl ester

| Composition of each tablet: | |
|---|---|
| - Salt of low molecular weight dermatan sulfate with tyrosine tert-butyl ester | 100 mg |
| - Maize starch | 46 mg |
| - Lactose | 40 mg |
| - Microgranular cellulose | 150 mg |
| - Polyvinylpyrrolidone CL | 50 mg |
| - Magnesium stearate | 5 mg |

Tablet coating:

| | |
|---|---|
| - Hydroxypropylmethylcellulose | 7 mg |
| - Polyethylene glycole 6000 | 0.3 mg |
| - Titanium dioxide | 2 mg |
| - Talc | 2 mg |
| - Polyvinylacetophthalate | 15 mg |
| - Diethylphthalate | 1.5 mg |
| - Stearic acid | 1.5 mg |

The tablets are prepared exactly as described in example 14.

The gastroresistant tablets containing 100 mg of the salts of the glycosaminoglycans with the esters of the aminoacids described in examples 1, 2, 3, 4, 5, 6, 7, 8, 9 and 11 are prepared in the same manner.

## Claims

### Claims for the following Contracting States : BE, DE, FR, GB, NL

1. Salts of glycosaminoglycans under acidic form with esters of aminoacids of formula

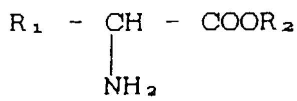

I

wherein $R_1$ represents hydrogen, straight or branched $C_1$-$C_6$-alkyl, aryl, substituted or unsubstituted arylalkyl and $R_2$ represents straight or branched $C_1$-$C_{12}$-alkyl, cycloalkyl, aryl or arylalkyl.

2. Salts according to claim 1 characterized in that the glycosaminoglycan is selected from heparin, fractions of heparin with molecular weights from 1500 to 8000 Daltons, glucuronylglycosaminoglycan sulfate, dermatan sulfate or fractions of dermatan sulfate with molecular weight from 2000 to 8000 Daltons, which the esters of aminoacids of formula I are selected from the methyl, ethyl, isopropyl, tert-butyl, isoamyl or benzyl esters of glycine, tyrosine or phenylalanine.

3. Process for the preparation of the salts according to each of claims 1 and 2 characterized in that an aqueous solution of an alkaline or alkaline-earth salt of a glycosaminoglycan is percolated through a chromatographic column containing a cationic resin activated under $H^+$ form and that the resulting eluate containing the glycosaminoglycan under acidic form is directly poured on an alcoholic solution of the ester of aminoacid of formula

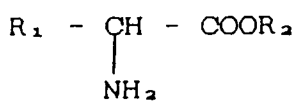

I

wherein $R_1$ and $R_2$ have the above seen meaning, the amount of the ester being equimolecular in respect to the acidic groups present in the glycosaminoglycan, obtaining a hydroalcoholic solution that is evaporated under vacuum to eliminate the alcoholic solvent and then is freeze-dried.

4. Process according to claim 3 characterized in that AG 50W X8 resin under $H^+$ form is the cationic resin and that methanol is the alcohol used to dissolve the ester of aminoacid of formula I.

5. Pharmaceutical formulations containing as active principle one of the salts according to each of claims 1 and 2.

6. Pharmaceutical formulations according to claim 5 characterized in that they are orally administrable.

7. Pharmaceutical formulations according to claim 6 characterized in that they are made by gastroresistant capsules or tablets containing from 50 to 500 mg of the salts as defined according to each of claims 1 and 2.

8. Use of the salts according to each of claims 1 and 2 for the production of a pharmaceutical composition for the prevention and treatment of the thrombotic and atherosclerotic pathologies.

### Claims for the following Contracting State : ES

1. Process for the preparation of salts of glycosaminoglycans with esters of aminoacids of formula

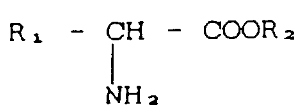

I

wherein $R_1$ represents hydrogen, straight or branched $C_1$-$C_6$-alkyl, aryl, substituted or unsubstituted arylalkyl and $R_2$ represents straight or branched $C_1$-$C_{12}$-alkyl, cycloalkyl, aryl or arylalkyl, characterized in that an aqueous solution of an alkaline or alkaline-earth salt of a glycosaminoglycan is percolated through a chromatographic column containing a cationic resin activated under $H^+$ form and that the resulting eluate containing the glycasaminoglycan under acidic form is directly poured on an alcoholic solution of the ester of aminoacid of formula

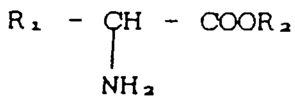

I

wherein $R_1$ and $R_2$ have the above seen meaning, the amount of the ester being equimolecular in respect to the acidic groups present in the glycosaminoglycan, obtaining a hydroalcoholic solution that is evaporated under vacuum to eliminate the alcoholic solvent and then is freeze-dried.

2. Process according to claim 1, characterized in that the glycosaminoglycan is selected from heparin, fractions of heparin with molecular weights from 1500 to 8000 Daltons, glucuronylglycosaminoglycan sulfate, dermatan sulfate or fractions of dermatan sulfate with molecular weights from 2000 to 8000 Daltons, while the esters of aminoacids of formula I are selected from the methyl, ethyl, isopropyl, tert-butyl, isoamyl or benzyl esters of glycine, tyrosine or phenylalanine.

3. Process according to claim 1, characterized in that AG 50W X8 resin under $H^+$ form is the cationic resin and that methanol is the alcohol used to dissolve the ester of aminoacid of formula I.

**Patentansprüche**

**Patentansprüche für folgende Vertragsstaaten : BE, DE, FR, GB, NL**

1. Salze von Glycosaminoglycanen in saurer Form mit Estern von Aminosäuren der Formel

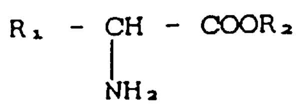

I

worin $R_1$ Wasserstoff, geradkettiges oder verzweigtkettiges $C_1$-$C_6$-Alkyl, Aryl, substituiertes oder unsubstituiertes Arylalkyl bedeutet und $R_2$ geradkettiges oder verzweigtkettiges $C_1$-$C_{12}$-Alkyl, Cycloalkyl, Aryl oder Arylalkyl bedeutet.

2. Salze nach Anspruch 1, dadurch gekennzeichnet, daß das Glycosaminoglycan ausgewählt wird aus Heparin, Fraktionen von Heparin mit Molekulargewichten von 1500 bis 8000 Dalton, Glucuronylglycosaminoglycansulfat, Dermatansulfat oder Fraktionen von Dermatansulfat mit Molekulargewichten von 2000 bis 8000 Dalton, während die Ester der Aminosäuren der Formel I ausgewählt werden aus Methyl-, Ethyl-, Isopropyl-, tert.-Butyl-, Isoamyl- oder Benzylestern des Glycins, Tyrosins oder Phenylalanins.

3. Verfahren zur Herstellung von Salzen nach einem der Ansprüche 1 und 2, dadurch gekennzeichnet, daß eine wäßrige Lösung eines Alkali- oder Erdalkalisalzes eines Glycosaminoglycans durch eine chromatographische Säule perkoliert wird, welche ein kationisches Harz, aktiviert in der $H^+$-Form, enthält und das entstehende Eluat, welches das Glycosaminoglycan in saurer Form enthält, direkt in einer alkoholischen Lösung des Esters der Aminosäure der Formel

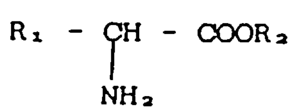

I

worin $R_1$ und $R_2$ die oben gegebenen Bedeutungen besitzen, gegeben wird, wobei die Menge an Ester äquimolar, bezogen auf die sauren Gruppen, die in dem Glycosaminoglycan vorhanden sind, ist, und eine wäßrig alkoholische

Lösung erhalten wird, die im Vakuum zur Entfernung des alkoholischen Lösungsmittels eingedampft und dann gefriergetrocknet wird.

4. Verfahren nach Anspruch 3, dadurch gekennzeichnet, daß AG 50W X8-Harz in $H^+$-Form als kationisches Harz verwendet wird und daß Methanol der Alkohol ist, der zur Auflösung des Esters der Aminosäure der Formel I verwendet wird.

5. Pharmazeutische Zubereitungen, enthaltend als aktiven Wirkstoff eines der Salze nach einem der Ansprüche 1 oder 2.

6. Pharmazeutische Zubereitungen nach Anspruch 5, dadurch gekennzeichnet, daß sie oral verabreichbar sind.

7. Pharmazeutische Zubereitungen nach Anspruch 6, dadurch gekennzeichnet, daß sie in Form gastroresistenter Kapseln oder Tabletten vorliegen, die 50 bis 500 mg der Salze, wie in einem Ansprüche 1 oder 2 definiert, enthalten.

8. Verwendung der Salze nach einem der Ansprüche 1 oder 2 zur Herstellung pharmazeutischer Zubereitungen für die Prophylaxe und Behandlung thrombotischer und atherosklerotischer Pathologien.

**Patentansprüche für folgenden Vertragsstaat : ES**

1. Verfahren zur Herstellung von Salzen von Glycosaminoglycanen mit Estern von Aminosäuren der Formel

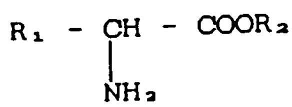

I

worin $R_1$ Wasserstoff, geradkettiges oder verzweigtkettiges $C_1$-$C_6$-Alkyl, Aryl, substituiertes oder unsubstituiertes Arylalkyl bedeutet und $R_2$ geradkettiges oder verzweigtkettiges $C_1$-$C_{12}$-Alkyl, Cycloalkyl, Aryl oder Arylalkyl bedeutet, dadurch gekennzeichnet, daß eine wäßrige Lösung eines Alkali- oder Erdalkalisalzes eines Glycosaminoglycans durch eine chromatographische Säule perkoliert wird, welche ein kationisches Harz, aktiviert in der $H^+$-Form, enthält und das entstehende Eluat, welches das Glycosaminoglycan in saurer Form enthält, direkt in einer alkoholischen Lösung des Esters der Aminosäure der Formel

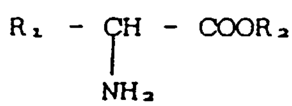

I

worin $R_1$ und $R_2$ die oben gegebenen Bedeutungen besitzen, gegeben wird, wobei die Menge an Ester äquimolar, bezogen auf die sauren Gruppen, die in dem Glycosaminoglycan vorhanden sind, ist, und eine wäßrig alkoholische Lösung erhalten wird, die im Vakuum zur Entfernung des alkoholischen Lösungsmittels eingedampft und dann gefriergetrocknet wird.

2. Verfahren nach Anspruch 1, dadurch gekennzeichnet, daß das Glycosaminoglycan ausgewählt wird aus Heparin, Fraktionen des Heparins mit Molekulargewichten von 1500 bis 8000 Dalton, Glucuronylglycosaminoglycansulfat, Dermatansulfat oder Fraktionen des Dermatansulfats mit Molekulargewichten von 2000 bis 8000 Dalton, während die Ester der Aminosäuren der Formel I ausgewählt werden aus Methyl-, Ethyl-, Isopropyl-, tert.-Butyl-, Isoamyl- oder Benzylestern von Glycin, Tyrosin oder Phenylalanin.

3. Verfahren nach Anspruch 1, dadurch gekennzeichnet, daß das AG 50W X8-Harz in $H^+$-Form als kationisches Harz verwendet wird und daß Methanol als Alkohol zur Auflösung des Esters der Aminosäure der Formel I verwendet wird.

## Revendications

**Revendications pour les Etats contractants suivants : BE, DE, FR, GB, NL**

1. Sels de glycosaminoglycanes sous forme acide, avec des esters d'acides aminés de formule :

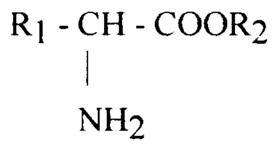

I

dans laquelle $R_1$ représente un atome d'hydrogène, un groupe alkyle $C_1$-$C_6$ linéaire ou ramifié, un groupe aryle, un groupe arylalkyle substitué ou non substitué, et $R_2$ représente un groupe alkyle $C_1$-$C_{12}$ linéaire ou ramifié, un groupe cycloalkyle, un groupe aryle, ou un groupe arylalkyle.

2. Sels selon la revendication 1, caractérisés en ce que le glycosaminoglycane est choisi parmi l'héparine, les fractions d'héparine de poids moléculaire compris entre 1.500 et 8.000 daltons, le glucuronylglycosaminoglycane sulfate, le dermatan sulfate, ou les fractions de dermatan sulfate de poids moléculaire compris entre 2.000 et 8.000 daltons, tandis que les esters d'acides aminés de formule (I) sont choisis parmi les esters méthyliques, éthyliques, isopropyliques, ter-butyliques, isoamyliques, ou benzyliques de la glycine, de la tyrosine, ou de la phénylalanine.

3. Procédé de préparation des sels selon chacune des revendications 1, et 2, caractérisé en ce qu'une solution aqueuse d'un sel alcalin ou alcalinoterreux d'un glycosaminoglycane est mise à percoler à travers une colonne de chromatographie contenant une résine cationique activée sous forme $H^+$, et que l'éluat ainsi obtenu, contenant le glycosaminoglycane sous forme acide, est versé directement dans une solution alcoolique de l'ester d'acide aminé de formule :

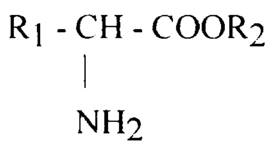

I

dans laquelle $R_1$ et $R_2$ sont tels que décrits plus haut, la quantité d'ester étant équimoléculaire par rapport aux groupements acide présents dans le glycosaminoglycane, pour donner une solution hydro-alcoolique qui est évaporée sous vide pour éliminer le solvant alcoolique, et qui est ensuite lyophilisée.

4. Procédé selon la revendication 3, caractérisé en ce que la résine AG 50W X8 sous forme $H^+$, est la résine cationique, et que le méthanol est l'alcool utilisé pour dissoudre l'ester d'acide aminé de formule (I).

5. Formulations pharmaceutiques contenant en tant que principe actif, un des sels selon chacune des revendications 1, et 2.

6. Formulations pharmaceutiques selon la revendication 5, caractérisées en ce qu'elles sont administrables par voie orale.

7. Formulations pharmaceutiques selon la revendication 6, caractérisées en ce qu'elles sont sous forme de gélules ou comprimés gastrorésistants contenant de 50 à 500 mg des sels tels que définis selon chacune des revendications 1, et 2.

8. Utilisation des sels selon chacune des revendications 1, et 2, pour la production d'une composition pharmaceutique destinée à la prévention et au traitement des pathologies thrombotiques, ou athérosclérotiques.

**Revendications pour l'Etat contractant suivant : ES**

1. Procédé de préparation de sels de glycosaminoglycanes avec des esters d'acides aminés de formule :

$$R_1 - \underset{\displaystyle NH_2}{\underset{|}{CH}} - COOR_2 \qquad I$$

dans laquelle $R_1$ représente un atome d'hydrogène, un groupe alkyle $C_1$-$C_6$ linéaire ou ramifié, un groupe aryle, un groupe arylalkyle substitué ou non substitué, et $R_2$ représente un groupe alkyle $C_1$-$C_{12}$ linéaire ou ramifié, un groupe cycloalkyle, un groupe aryle, ou un groupe arylalkyle, caractérisé en ce qu'une solution aqueuse d'un sel alcalin ou alcalinoterreux d'un glycosaminoglycane est mise à percoler à travers une colonne de chromatographie contenant une résine cationique activée sous forme $H^+$, et que l'éluat ainsi obtenu, contenant le glycosaminoglycane sous forme acide, est versé directement dans une solution alcoolique de l'ester d'acide aminé de formule :

$$R_1 - \underset{\displaystyle NH_2}{\underset{|}{CH}} - COOR_2 \qquad I$$

dans laquelle $R_1$ et $R_2$ sont tels que décrits plus haut, la quantité d'ester étant équimoléculaire par rapport aux groupements acide présents dans le glycosaminoglycane, pour donner une solution hydro-alcoolique qui est évaporée sous vide pour éliminer le solvant alcoolique, et qui est ensuite lyophilisée.

2. Procédé selon la revendication 1, caractérisé en ce que le glycosaminoglycane est choisi parmi l'héparine, les fractions d'héparine de poids moléculaire compris entre 1.500 et 8.000 daltons, le glucuronylglycosaminoglycane sulfate, le dermatan sulfate, ou les fractions de dermatan sulfate de poids moléculaire compris entre 2.000 et 8.000 daltons, tandis que les esters d'acides aminés de formule (I) sont choisis parmi les esters méthyliques, éthyliques, isopropyliques, ter-butyliques, isoamyliques, ou benzyliques de la glycine, de la tyrosine, ou de la phénylalanine.

3. Procédé selon la revendication 1, caractérisé en ce que la résine AG 50W X8 sous forme $H^+$, est la résine cationique, et que le méthanol est l'alcool utilisé pour dissoudre l'ester d'acide aminé de formule (I).